# EUROPÄISCHE PATENTSCHRIFT

(11) **EP 4 475 802 B1**
(45) Veröffentlichungstag und Bekanntmachung des Hinweises auf die Patenterteilung: **17.12.2025**
(21) Anmeldenummer: 23705335.0
(22) Anmeldetag: 31.01.2023
(51) Int. Cl.: A61F 5/01, A61F 5/30

(54) **PELOTTE ZUR AUFNAHME EINER BANDAGE UND BANDAGE**
PAD FOR RECEIVING A BANDAGE, AND BANDAGE
COUSSINET DESTINÉ À ÊTRE PLACÉ DANS UN BANDAGE ET BANDAGE

(30) Priorität: 10.02.2022 DE 202022100765 U
(43) Veröffentlichungstag der Anmeldung: 18.12.2024
(73) Patentinhaber: Thuasne Deutschland GmbH, 30539 Hannover (DE)
(72) Erfinder: SCHNEIDER-NIESKENS, Reinhold, 29223 Celle (DE)
(74) Vertreter: Jabbusch, Matthias
(86) Internationale Anmeldenummer: PCT/EP2023/052288
(87) Internationale Veröffentlichungsnummer: WO 2023/151985

(56) Entgegenhaltungen:
- EP-B1- 1 595 675
- DE-A1- 102012 105 626
- DE-A1- 4 439 088
- DE-U1- 202014 003 965
- US-A1- 2017 325 990

## Beschreibung

Die Erfindung betrifft eine Pelotte, die dazu vorgesehen ist, in einer Bandage aufgenommen zu werden und die zur Abstützung des Körpergewebes vorgesehen ist und auf der Seite, die zur Ausrichtung auf den Körper vorgesehen ist, Lüftungskanäle aufweist. Weiterhin betrifft die Erfindung eine Bandage.

Eine solche Pelotte ist aus der DE 20 2014 003 965 U1 bekannt. Hier ist eine Pelotte beschrieben, die eine Basis aufweist, wobei an der Basis in regelmäßiger Anordnung Anlagekörper ausgebildet sind, die jeweils eine dreieckige Anlagefläche aufweisen. Zwischen den Anlagekörpern sind Lüftungskanäle ausgebildet, die sich zwischen zwei einander gegenüberliegenden Rändern der Pelotte erstrecken und sich in einem Winkel von jeweils 60° kreuzen. In den Kreuzungsbereichen der Lüftungskanäle sind in der Basis Durchbrüche angeordnet, die ein Abdampfen von Flüssigkeit, insbesondere Körperschweiß, ermöglichen, die sich zwischen abzustützendem Körpergewebe und Anlageflächen bildet.

Eine weitere Pelotte ist aus der DE 44 39 088 A1 bekannt. Bei dieser sind die Anlagekörper quadratisch ausgebildet. Insgesamt wird eine möglichst druckfreie Abstützung erreicht. Durch die Trennung der Anlagekörper durch schluchtartige Einschnitte wird eine hohe Flächenelastizität erreicht, wodurch eine möglichst druckspitzenfreie Abstützung der Gliedmaßen und Extremitäten erreicht wird.

Der Erfindung liegt die Aufgabe zugrunde, eine Pelotte und eine Bandage der eingangs genannten Art zu schaffen, die eine besonders hohe Elastizität aufweisen.

Die Lösung dieser Aufgabe erfolgt mit einer Pelotte mit den Merkmalen des Schutzanspruchs 1 sowie mit einer Bandage mit den Merkmalen des Schutzanspruchs 14. Weiterbildungen und vorteilhafte Ausgestaltungen der Erfindungen sind in den jeweils nachgeordneten Schutzansprüchen beschrieben.

Die Pelotte, die dazu vorgesehen ist, in einer Bandage aufgenommen zu werden und die zur Abstützung des Körpergewebes vorgesehen ist und auf der Seite, die zur Ausrichtung auf den Körper vorgesehen ist, Lüftungskanäle aufweist, zeichnet sich erfindungsgemäß dadurch aus, dass die Pelotte auf der Seite, die dazu vorgesehen ist, die vom Körper abgewandte Seite zu bilden, Außenkanäle aufweist. Durch ein solches Miteinander von Lüftungskanälen auf der Innenseite und Außenkanälen auf der Außenseite wird die Dicke der Pelotte in Teilbereichen reduziert, so dass eine besonders hohe Elastizität und Dehnbarkeit der Pelotte erreicht wird. Die Lüftungskanäle auf der Innenseite dienen dabei insbesondere auch zusätzlich dazu, Feuchtigkeit, insbesondere Körperschweiß, abzuführen so dass ein hoher Tragekomfort erreicht ist.

Die Dehnungseigenschaften der Bandage werden weiterhin dadurch verbessert, dass die Außenkanäle in ungefähr 90° zur Umfangsrichtung der Bandage ausgerichtet sind.

In einer bevorzugten Ausgestaltung der Erfindung sind die Lüftungskanäle und die Außenkanäle parallel zueinander ausgebildet. Durch eine solche Anordnung wird die Dehnbarkeit besonders gut erreicht. Weiterhin ist es bevorzugt, dass die Lüftungskanäle und die Außenkanäle abwechselnd zueinander angeordnet sind. Dies lässt sich auch als Ziehharmonika-Struktur bezeichnen. Bei einer solchen Anordnung bleibt die Dicke der Pelotte insgesamt vergleichsweise dünn und es sind abwechselnd einmal die Lüftungskanäle auf der Innenseite und die Außenkanäle auf der Außenseite vorhanden. Insbesondere durch eine solche Anordnung lässt sich eine Dehnbarkeit im rechten Winkel zur Ausrichtung der Außenkanäle erreichen.

Bevorzugt sind die Lüftungskanäle und die Außenkanäle etwa gleich breit. Dadurch wird die Effektivität der vorher beschriebenen Ziehharmonika-Struktur besonders gut erreicht.

Weiterhin ist es bevorzugt, dass Durchbrüche in der Pelotte vorhanden sind. Diese sind bevorzugt in den Lüftungskanälen vorhanden, so dass Flüssigkeit von der Körperseite nach außen abgeführt werden kann. Die Lüftungskanäle weisen dabei bevorzugt eine erste Gruppe von parallel zueinander ausgerichteten Lüftungskanälen und eine zweite Gruppe von Lüftungskanälen auf, die rechtwinklig zu der ersten Gruppe von Lüftungskanälen ausgerichtet ist. In alternative Ausführungsformen kann es auch günstig sein, dass die erste Gruppe von Lüftungskanälen und die zweite Gruppe von Lüftungskanälen sich unter einem Winkel von plus minus 45° oder auch unter einem Winkel von plus minus 60°kreuzen.

In den Kreuzungsbereichen der ersten Gruppe der Lüftungskanäle und der zweiten Gruppe der Lüftungskanäle sind dann bevorzugt die Durchbrüche angeordnet, durch die Flüssigkeit über die Verdunstung nach außen gelangen kann. In einer besonders bevorzugten Ausgestaltung der Erfindung münden die Durchbrüche außenseitig zwischen den Außenkanälen. Außenseitig sind zwischen den Außenkanälen Wandbereiche vorhanden, in denen die Lüftungskanäle münden.

Die geometrische Ausgestaltung ist bevorzugt so, dass die Durchbrüche ein Raster ausbilden, das aus Eckpunkten von Quadraten gebildet ist. Durch eine solch gleichmäßige Verteilung wird eine zuverlässige Ableitung der Flüssigkeit gewährleistet.

Bevorzugt besteht die Pelotte aus einem Elastomer, insbesondere aus Silikon. Hier kann ein Silikonkautschuk oder auch ein TPU- oder ein TPE-Material verwendet werden.

In einer speziellen Ausführungsform ist die Pelotte ringförmig ausgebildet. Eine derartige Pelotte dient insbesondere zur Anwendung im Bereich des Knies und der Patellasehne.

In einer anderen Ausführungsform ist die Pelotte länglich ausgebildet und in einem Endbereich ringförmig ausgebildet beziehungsweise weist in einem Endbereich eine kreis- oder ovalförmige Ausnehmung auf. Eine derartige Pelotte ist zur Anwendung im Bereich des Ellenbogens vorgesehen.

In einer weiteren bevorzugten Ausführungsform ist die Pelotte "J"-förmig ausgebildet. Eine derartige Pelotte ist insbesondere zur Anwendung im Bereich der Knöchel vorgesehen.

Ein weiterer Aspekt der Erfindung besteht in der Bereitstellung einer Bandage, wobei die Bandage erfindungsgemäß einer oben beschriebenen Pelotte aufweist. Die Pelotte ist in ein dieses umgebende Textil eingearbeitet. Auf diese Weise wird eine erfindungsgemäße Bandage bereitgestellt.

Nachfolgend wird die Erfindung in der Zeichnung dargestellt. Aus den dort dargestellten und beschriebenen Ausführungsbeispielen der Erfindung können sich weitere erfinderische Merkmale ergeben.
- Figur 1:: Eine Kniepelotte mit Figur 1a, einer körperseitigen Ansicht; Figur 1b einer vom Körper abgewandten Ansicht und Figur 1c einer geschnittenen perspektivischen Seitenansicht;
- Figur 2:: Eine flächige Pelotte mit Figur 2a, einer körperseitigen Ansicht und Figur 2b einer vom Körper abgewandten Ansicht;
- Figur 3:: Querschnitt durch den oberen Teil einer Patella-Pelotte mit Figur 3a einer ungedehnten Patella-Pelotte und Figur 3b einer gedehnten Patella-Pelotte;
- Figur 4:: Eine "J"-förmige Pelotte zur Anwendung im Knöchelbereich mit Figur 4a, einer Ansicht der Pelotte, die zum Körper ausgerichtet ist; Figur 4b einer Ansicht der Seite der Pelotte, die vom Körper abgewandt ist und Figur 4c einer geschnittenen perspektivischen Ansicht der Pelotte; und
- Figur 5:: Figur 5a, eine perspektivische geschnittene Ansicht einer länglichen Pelotte zur Darstellung der Ziehharmonika-Struktur, die in einem Endbereich ringförmig ausgebildet ist und Figur 5b eine perspektivische geschnittene Ansicht durch den Belüftungs-Querkanal mit den Belüftungsbohrungen einer länglichen Pelotte.

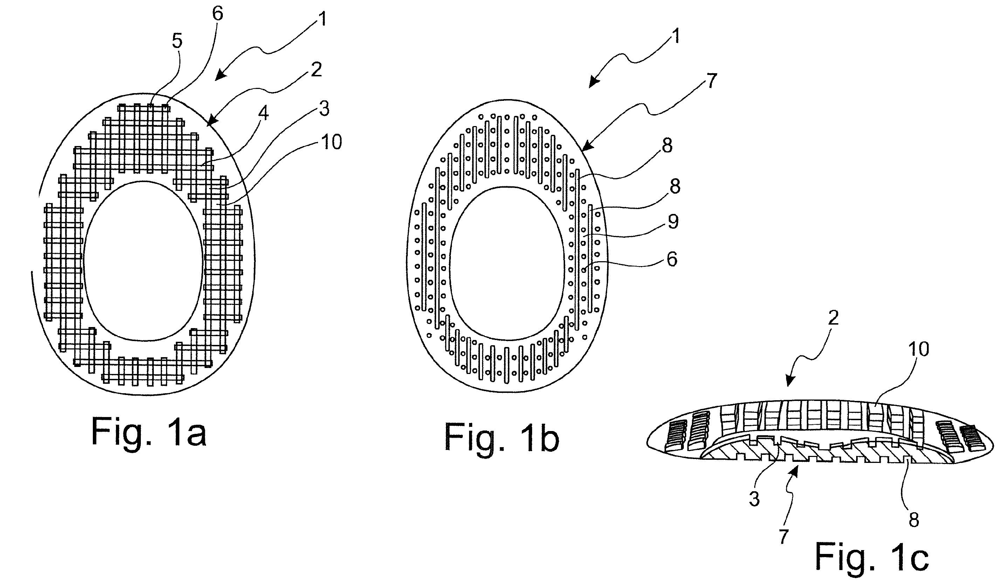

In Figur 1 ist eine Pelotte 1 dargestellt, die hier als ringförmige Pelotte ausgebildet ist, die zur Abstützung im Kniebereich und der Patellasehne vorgesehen ist. In Figur 1a ist die Innenseite 2, also die dem Körper zugewandte Seite der Pelotte 1 zu sehen. Auf der Innenseite 2 sind Lüftungskanäle ausgebildet, wobei hier eine erste Gruppe von Lüftungskanälen 3 und eine zweite, im rechten Winkel dazu verlaufende Gruppe von Lüftungskanälen 4 vorgesehen ist, so dass zwischen den Lüftungskanälen jeweils quadratische Anlagekörper 10 ausgebildet sind. In den Kreuzungsbereichen 5 der ersten Gruppe der Lüftungskanäle 3 und der zweiten Gruppe der Lüftungskanäle 4 sind Durchbrüche 6 ausgebildet, die dazu vorgesehen und ausgebildet sind, Flüssigkeit, insbesondere Körperschweiß, über Verdunstung von innen nach außen zu transportieren.

In Figur 1b ist die Pelotte 1 aus Figur 1a von der anderen Seite, nämlich von der Außenseite 7, dargestellt. Diese Außenseite 7 ist dazu vorgesehen und ausgelegt, auf der vom Körper abgewandten Seite getragen zu werden. In der Außenseite 7 sind Außenkanäle 8 ausgebildet. Diese sind parallel zueinander ausgerichtet und im Ergebnis parallel zu der ersten Gruppe der Lüftungskanäle 3 auf der Innenseite 2 der Pelotte 1 ausgerichtet. Zwischen den Außenkanälen 8 sind Wandbereiche 9 vorhanden. In diesen Wandbereichen 9 münden die Durchbrüche 6, die sich von der Innenseite 2 zur Außenseite 7 erstrecken.

In Figur 1c ist die Pelotte 1 in einer geschnittenen perspektivischen Ansicht dargestellt. Hier ist insbesondere zu erkennen, dass die Lüftungskanäle 3 der ersten Gruppe auf der Innenseite 2 der Pelotte parallel und versetzt zu den Außenkanälen 8 der Pelotte 1 verlaufen. Dadurch ergibt sich die Ziehharmonika-Struktur, durch die eine besonders hohe Elastizität erreicht wird. Weiterhin sind hier auch die quadratischen Anlagekörper 10 zu erkennen.

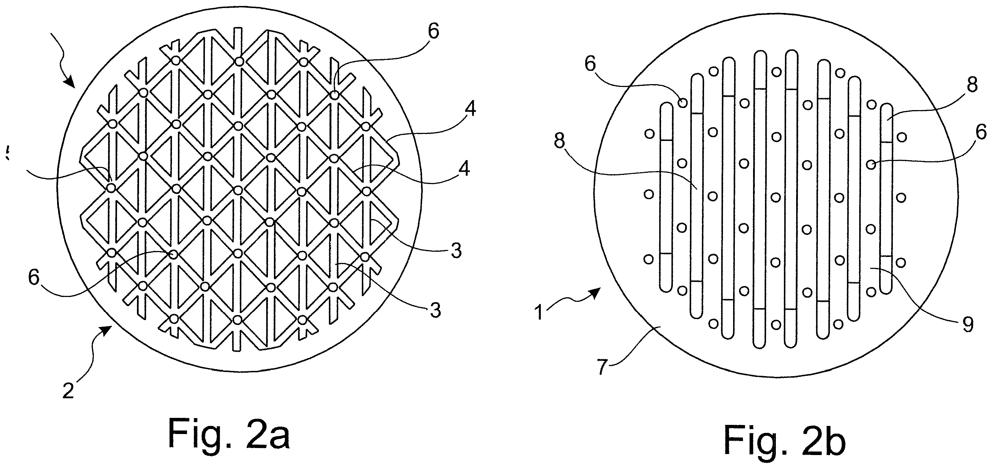

In Figur 2 ist eine flächige Pelotte 1 dargestellt, wobei in 2a die Innenseite 2 dargestellt ist, also die dem Körper zugewandte Seite der Pelotte. Auf der Innenseite 2 sind Lüftungskanäle 3 vorgesehen, die sich hier unter Winkeln von 90° und 45° kreuzen. In den Kreuzungsbereichen 5 sind Durchbrüche 6 vorgesehen. Die Lüftungskanäle können auch hier als zwei Gruppen von Lüftungskanälen beschrieben werden. Eine erste Gruppe von Lüftungskanälen 3 verläuft parallel zueinander. Eine zweite Gruppe von Lüftungskanälen 4 verläuft ebenfalls parallel zueinander. Die beiden Gruppen von Lüftungskanälen schneiden sich unter einem Winkel von 45°, so dass jeweils Dreiecke gebildet werden, die einen 90°-Winkel und zwei 45°-Winkel aufweisen.

Auf der in der Figur 2b dargestellten Rückseite der Pelotte ist die Außenseite 7 dargestellt. Diese Außenseite 7 ist dazu vorgesehen und ausgelegt, auf der vom Körper abgewandten Seite getragen zu werden. In der Außenseite 7 sind Außenkanäle 8 ausgebildet. Diese sind parallel zu der ersten Gruppe der Lüftungskanäle 3 auf der Innenseite 2 der Pelotte 1 ausgerichtet. Zwischen den Außenkanälen 8 sind Wandbereiche 9 vorhanden. In diesen Wandbereichen 9 münden die Durchbrüche 6, die sich von der Innenseite 2 zur Außenseite 7 erstrecken.

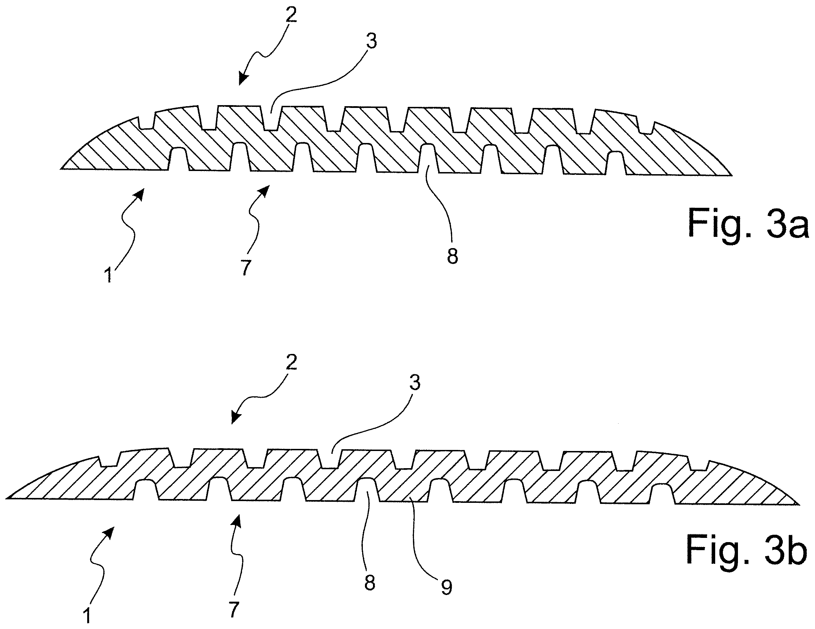

In Figur 3 ist noch einmal ein Querschnitt durch eine Patella-Pelotte gemäß Figur 1 dargestellt. Die obere Seite ist die Innenseite 2, die körperseitig getragen wird. Die untere Seite ist die Außenseite 7, die auf der vom Körper abgewandten Seite getragen wird. Auf der Innenseite 2 sind die Lüftungskanäle 3 und auf der Außenseite 7 die Außenkanäle 8 zu erkennen. Durch die Kanäle entsteht eine Ziehharmonikastruktur, so dass die Pelotte insgesamt gedehnt werden kann. Dies wir in den Figuren 3a und 3b besonders deutlich.

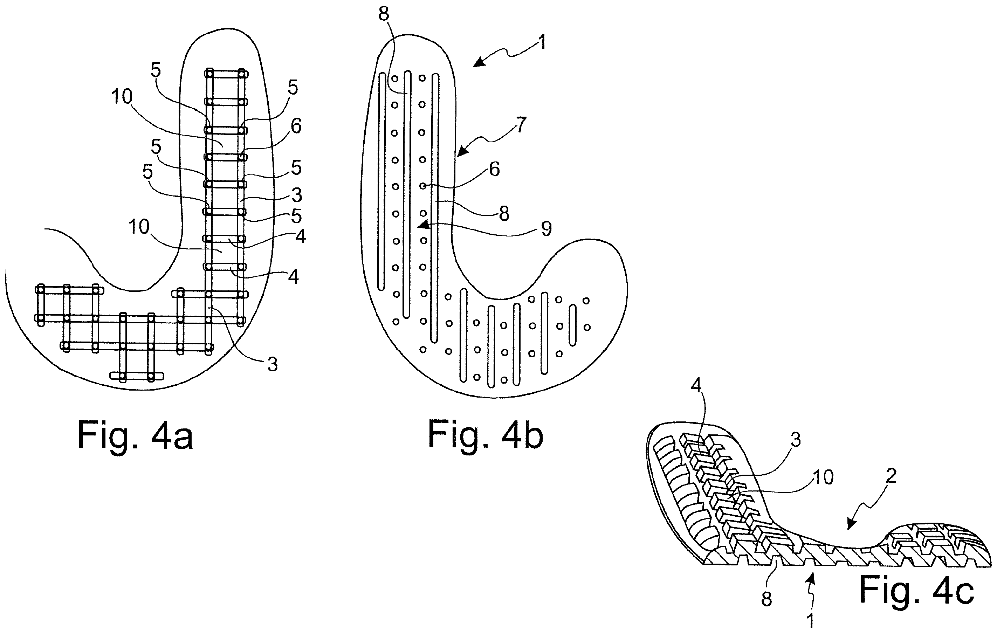

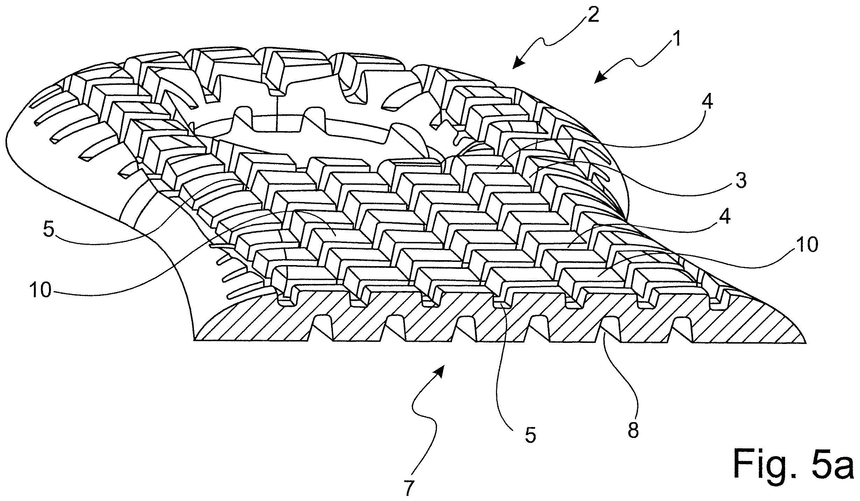

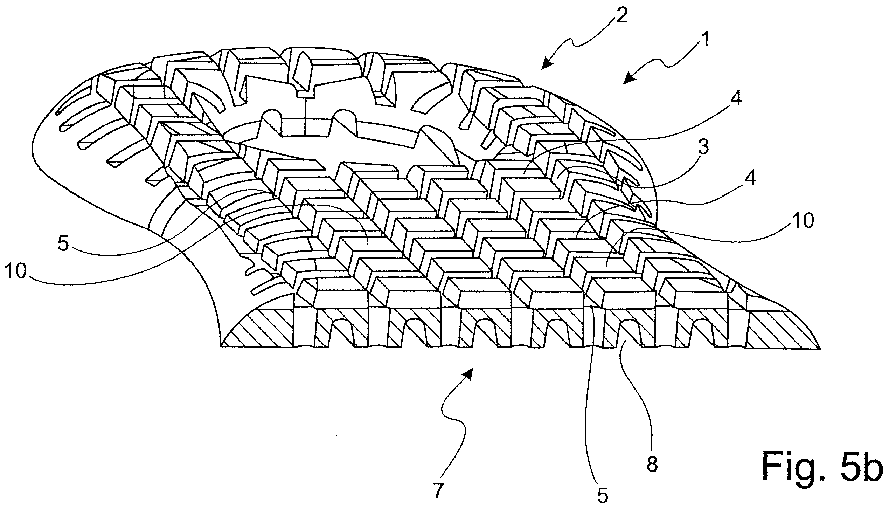

In Figur 4 mit den Figuren 4a, 4b und 4c ist eine "J"-förmige Pelotte dargestellt, die zur Abstützung des Knöchels dient. In Figur 4a ist dabei die Seite dargestellt, die körperseitig ausgerichtet ist, in Figur 4b ist die Seite dargestellt, die vom Körper abgewandt ist und in Figur 5a ist eine geschnittene perspektivische Ansicht dargestellt. In Figur 5b ist ein Schnitt vergleichbar zur Figur 5a zur Verdeutlichung der Ziehharmonikastruktur. Vergleichbar zur Pelotte gemäß Figur 1 ist in der Figur 4a eine erste Gruppe von Lüftungskanälen 3 und eine zweite Gruppe von Lüftungskanälen 4 ausgebildet, die in einem rechten Winkel zueinander verlaufen und sich in Kreuzungsbereichen 5 treffen. Die Kreuzungsbereiche 5 bilden dabei ein quadratisches Raster aus. In den Kreuzungsbereichen 5 sind auch jeweils die Durchbrüche 6 vorgesehen, durch die Flüssigkeit von innen nach außen gelangen kann. Zwischen diesen Kreuzungsbereichen 5 sind die Anlagekörper 10 ausgebildet.

In Figur 4b ist die Außenseite 7 der Pelotte 1 dargestellt. Hier sind die Außenkanäle 8 vorhanden, wobei zwischen den Außenkanälen 8 Wandbereiche 9 vorhanden sind. In den Wandbereichen 9 münden die Durchbrüche 6. In der perspektivischen Ansicht gemäß Figur 4c ist oben die Innenseite 2 und unten die Außenseite 7 zu sehen. Auf der Innenseite 2 ist das Raster aus Lüftungskanälen 3 der ersten Gruppe und Lüftungskanälen 4 der zweiten Gruppe zu erkennen, zwischen denen die Anlagekörper 10 ausgebildet sind. Auf der Außenseite 7 sind Außenkanäle 8 vorhanden, die parallel und versetzt zu der ersten Gruppe der Lüftungskanäle 3 ausgebildet sind. Dadurch ergibt sich die Ziehharmonika-Struktur, durch die eine besonders hohe Elastizität erreicht wird.

In Figur 5 ist eine geschnittene perspektivische Ansicht einer Pelotte 1 dargestellt, die zur Behandlung der Sehnen im Bereich des Ellenbogens vorgesehen ist. Diese Pelotte ist länglich und weist in einem Endbereich eine Ausnehmung auf, die annähernd kreisförmig oder leicht oval ist. Auf der Innenseite 2 weist diese Pelotte auch eine erste Gruppe von Lüftungskanälen 3 auf, die sich in der dargestellten Figur von oben nach unten erstrecken und eine zweite Gruppe von Lüftungskanälen 4, auf die sich in der dargestellten Figur von rechts nach links erstrecken. Zwischen dieser ersten Gruppe von Lüftungskanälen 3 und der zweiten Gruppe von Lüftungskanälen 4 befinden sich die Anlagekörper 10. In den Kreuzungsbereichen 5 sind die Durchbrüche 6 vorgesehen. Auf der Außenseite 7 der Pelotte 1 sind Außenkanäle 8 vorgesehen, die parallel und alternierend zu den Lüftungskanälen 3 der ersten Gruppe der Lüftungskanäle vorhanden sind. Dadurch wird insbesondere eine seitliche Elastizität der Pelotte 1 erreicht.

## Patentansprüche

1. Pelotte (1), die dazu vorgesehen ist in einer Bandage aufgenommen zu werden, und die zur Abstützung des Körpergewebes vorgesehen ist und auf der Seite (12), die zur Ausrichtung auf den Körper vorgesehen ist, Lüftungskanäle (3,4) aufweist,
**dadurch gekennzeichnet,**
**dass** die Pelotte (1) auf der Seite, die dazu vorgesehen ist, die vom Körper abgewandte Seite zu bilden, Außenkanäle (8) aufweist.

2. Pelotte nach Anspruch 1, **dadurch gekennzeichnet, dass** die Außenkanäle (8) ungefähr 90° zur Umfangsrichtung der Bandage ausgerichtet sind.

3. Pelotte nach Anspruch 1 oder 2, **dadurch gekennzeichnet, dass** die Lüftungskanäle (3) und die Außenkanäle (8) parallel zueinander ausgebildet sind.

4. Pelotte nach einem der vorhergehenden Ansprüche, **dadurch gekennzeichnet, dass** die Lüftungskanäle (3) und die Außenkanäle (8) abwechselnd zueinander angeordnet sind.

5. Pelotte nach einem der vorhergehenden Ansprüche, **dadurch gekennzeichnet, dass** die Lüftungskanäle (3) und die Außenkanäle (8) etwa gleich breit sind.

6. Pelotte nach einem der vorhergehenden Ansprüche, **dadurch gekennzeichnet, dass** Durchbrüche (6) in der Pelotte (1) vorhanden sind.

7. Pelotte nach einem der vorhergehenden Ansprüche, **dadurch gekennzeichnet, dass** die Lüftungskanäle eine erste Gruppe von parallel zueinander ausgerichteten Lüftungskanälen (3) ausbilden und eine zweite Gruppe von Lüftungskanälen (4) ausbilden, die rechtwinklig zu der ersten Gruppe von Lüftungskanälen (3) ausgerichtet ist.

8. Pelotte nach einem der Ansprüche 1 bis 6, **dadurch gekennzeichnet, dass** die Lüftungskanäle eine erste Gruppe von parallel zueinander ausgerichteten Lüftungskanälen (3) und eine zweite Gruppe von Lüftungskanälen (4) ausbilden, die in plus minus 45° oder auch plus minus 60° zu der ersten Gruppe von Lüftungskanälen (3) ausgerichtet ist.

9. Pelotte nach einem der Ansprüche 7 oder 8, **dadurch gekennzeichnet, dass** die Durchbrüche (6) in den Kreuzungsbereichen (5) der ersten Gruppe der Lüftungskanäle (3) und der zweiten Gruppe der Lüftungskanäle (4) vorgesehen sind.

10. Pelotte nach einem der Ansprüche 6 bis 9, **dadurch gekennzeichnet, dass** die Durchbrüche (6) außenseitig zwischen den Außenkanälen (3, 4) münden.

11. Pelotte nach einem der Ansprüche 6 bis 9, **dadurch gekennzeichnet, dass** die Durchbrüche (6) ein Raster ausbilden, das aus Eckpunkten von Quadraten gebildet ist.

12. Pelotte nach einem der vorhergehenden Ansprüche, **dadurch gekennzeichnet, dass** die Pelotte (1) aus einem Elastomer besteht.

13. Pelotte nach einem der vorhergehenden Ansprüche, **dadurch gekennzeichnet, dass** die Pelotte (1) ringförmig ausgebildet ist.

14. Pelotte nach einem der Ansprüche 1 bis 12, **dadurch gekennzeichnet, dass** die Pelotte (1) länglich ausgebildet ist und in einem Endbereich ringförmig ausgebildet ist.

15. Pelotte nach einem der Ansprüche 1 bis 12, **dadurch gekennzeichnet, dass** die Pelotte "J"-förmig ausgebildet ist.

16. Bandage,
**dadurch gekennzeichnet,**
**dass** die Bandage eine Pelotte (1) nach einem der vorhergehenden Ansprüche aufweist.

17. Bandage nach Anspruch 16, **dadurch gekennzeichnet, dass** die Pelotte (1) in ein diese umgebendes Textil eingearbeitet ist.

## Claims

1. A pad (1), which is provided to be received in a bandage, and which is provided for supporting the body tissue and which has ventilation channels (3,4) on the side (12) which is provided to be oriented towards the body,
**characterized in that**
the pad (1) has outer channels (8) on the side which is provided to form the side facing away from the body.

2. The pad according to Claim 1, **characterized in that** the outer channels (8) are aligned approximately 90° to the circumferential direction of the bandage.

3. The pad according to Claim 1 or 2, **characterized in that** the ventilation channels (3) and the outer channels (8) are formed parallel to one another.

4. The pad according to one of the preceding claims, **characterized in that** the ventilation channels (3) and the outer channels (8) are arranged alternately with respect to one another.

5. The pad according to one of the preceding claims, **characterized in that** the ventilation channels (3) and the outer channels (8) have approximately the same width.

6. The pad according to one of the preceding claims, **characterized in that** apertures (6) are present in the pad (1).

7. The pad according to one of the preceding claims, **characterized in that** the ventilation channels form a first group of ventilation channels (3) aligned parallel to one another, and form a second group of ventilation channels (4), which is aligned at right angles to the first group of ventilation channels (3).

8. The pad according to one of Claims 1 to 6, **characterized in that** the ventilation channels form a first group of ventilation channels (3) aligned parallel to one another, and a second group of ventilation channels (4), which is aligned at plus minus 45° or also plus minus 60° to the first group of ventilation channels (3).

9. The pad according to one of Claims 7 or 8, **characterized in that** the apertures (6) are provided in the crossing regions (5) of the first group of the ventilation channels (3) and of the second group of the ventilation channels (4).

10. The pad according to one of Claims 6 to 9, **characterized in that** the apertures (6) open out outside between the outer channels (3,4).

11. The pad according to one of Claims 6 to 9, **characterized in that** the apertures (6) form a grid which is formed from corner points of squares.

12. The pad according to one of the preceding claims, **characterized in that** the pad (1) consists of an elastomer.

13. The pad according to one of the preceding claims, **characterized in that** the pad (1) is configured in an annular manner.

14. The pad according to one of Claims 1 to 12, **characterized in that** the pad (1) is configured in an elongate manner and is configured in an annular manner in an end region.

15. The pad according to one of Claims 1 to 12, **characterized in that** the pad is configured in a "J"-shaped manner.

16. A bandage,
**characterized in that**
the bandage has a pad (1) according to one of the preceding claims.

17. The bandage according to Claim 16, **characterized in that** the pad (1) is worked into a textile surrounding it.

## Revendications

1. Coussinet (1) qui est destiné à être placé dans un bandage et qui est prévu pour soutenir du tissu corporel et qui comporte des conduits d'aération (3, 4) sur le côté (12), qui est prévu pour orientation sur le corps,
**caractérisé en ce que**
le coussinet (1) comporte des conduits extérieurs (8) sur le côté, qui est prévu pour former le côté éloigné du corps.

2. Coussinet selon la revendication 1, **caractérisé en ce que** les conduits extérieurs (8) sont orientés à peu près à 90° par rapport au sens périphérique du bandage.

3. Coussinet selon la revendication 1 ou 2, **caractérisé en ce que** les conduits d'aération (3) et les conduits extérieurs (8) sont constitués parallèles les uns aux autres.

4. Coussinet selon l'une quelconque des revendications précédentes, **caractérisé en ce que** les conduits d'aération (3) et les conduits extérieurs (8) sont disposés en alternance les uns par rapport aux autres.

5. Coussinet selon l'une quelconque des revendications précédentes, **caractérisé en ce que** les conduits d'aération (3) et les conduits extérieurs (8) sont à peu près de la même largeur.

6. Coussinet selon l'une quelconque des revendications précédentes, **caractérisé en ce que** des passages (6) sont présents dans le coussinet (1).

7. Coussinet selon l'une quelconque des revendications précédentes, **caractérisé en ce que** les conduits d'aération constituent un premier groupe de conduits d'aération (3) orientés parallèlement les uns aux autres et constituent un deuxième groupe de conduits d'aération (4), qui est orienté à angle droit par rapport au premier groupe de conduits d'aération (3).

8. Coussinet selon l'une quelconque des revendications 1 à 6, **caractérisé en ce que** les conduits d'aération constituent un premier groupe de conduits d'aération (3) orientés parallèlement les uns aux autres et un deuxième groupe de conduits d'aération (4), qui est orienté à plus ou moins 45° ou également à plus ou moins 60° par rapport au premier groupe de conduits d'aération (3).

9. Coussinet selon l'une quelconque des revendications 7 ou 8, **caractérisé en ce que** les passages (6) sont prévus dans les zones de croisement (5) du premier groupe des conduits d'aération (3) et du deuxième groupe des conduits d'aération (4).

10. Coussinet selon l'une quelconque des revendications 6 à 9, **caractérisé en ce que** les passages (6) débouchent extérieurement entre les conduits extérieurs (3, 4).

11. Coussinet selon l'une quelconque des revendications 6 à 9, **caractérisé en ce que** les passages (6) constituent une trame, qui est formée de points d'angle de carrés.

12. Coussinet selon l'une quelconque des revendications précédentes, **caractérisé en ce que** le coussinet (1) est composé d'un élastomère.

13. Coussinet selon l'une quelconque des revendications précédentes, **caractérisé en ce que** le coussinet (1) est constitué de forme annulaire.

14. Coussinet selon l'une quelconque des revendications 1 à 12, **caractérisé en ce que** le coussinet (1) est constitué en sens longitudinal et est constitué de forme annulaire dans une zone d'extrémité.

15. Coussinet selon l'une quelconque des revendications 1 à 12, **caractérisé en ce que** le coussinet est constitué en «forme de J».

16. Bandage,
**caractérisé en ce que**
le bandage comporte un coussinet (1) selon l'une quelconque des revendications précédentes.

17. Bandage selon la revendication 16, **caractérisé en ce que** le coussinet (1) est intégré dans un textile entourant celui-ci.
